Europäisches Patentamt

European Patent Office

Office européen des brevets

(19)

(11) Veröffentlichungsnummer: **0 148 724**
**B1**

(12) **EUROPÄISCHE PATENTSCHRIFT**

(45) Veröffentlichungstag der Patentschrift:
**01.06.88**

(51) Int. Cl.⁴: **A 61 K 31/565**

(21) Anmeldenummer: **84730146.2**

(22) Anmeldetag: **18.12.84**

(54) **Mehrstufenkombinationspräparat und seine Verwendung zur oralen Kontrazeption.**

(30) Priorität: **22.12.83 DE 3347125**

(43) Veröffentlichungstag der Anmeldung:
**17.07.85 Patentblatt 85/29**

(45) Bekanntmachung des Hinweises auf die Patenterteilung:
**01.06.88 Patentblatt 88/22**

(84) Benannte Vertragsstaaten:
**AT BE CH DE FR GB IT LI LU NL SE**

(56) Entgegenhaltungen:
**EP - A - 0 050 867**
**DE - A - 2 431 704**
**DE - A - 3 341 638**

**B. Helwig: Moderne Arzneimittel, Stuttgart 1980, Seiten 1406-1407**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen,**
**Müllerstrasse 170/178 Postfach 65 03 11,**
**D-1000 Berlin 65 (DE)**

(72) Erfinder: **Lachnit-Fixson, Ursula, Dr., Schleinitzstr. 10,**
**D-1000 Berlin 33 (DE)**
Erfinder: **Unger, Renate, Holtzendorffstrasse 19,**
**D-1000 Berlin (DE)**

## Beschreibung

Die Erfindung betrifft ein Zwei- bzw. Dreistufenkombinationspräparat aus 21 bzw. 28 Applikationseinheiten für die tägliche Applikation einer Einheit und seine Verwendung zur oralen Kontrazeption.

Mehrstufenkombinationspräparate zur oralen Kontrazeption sind bereits bekannt, beispielsweise aus DE-A 2 365 103 und den daraus abgeleiteten Patenten. Diese Mehrstufenpräparate bestehen aus 21 bzw. 28 Dragées und enthalten in der ersten Stufe 4–6 Dragées, in der jedes Dragée eine Östrogenmenge enthält, die der von 0,02–0,05 mg Ethinylöstradiol entspricht, und eine Gestagenmenge, die der von 0,04–0,09 mg d-Norgestrel entspricht, und in der zweiten Stufe 4–6 Dragées, die jeweils die 1- bis 2fache Östrogenmenge der ersten Stufe enthalten, beispielsweise 0,03–0,05 mg Ethinylöstradiol, und die 1- bis 1,5fache Gestagenmenge der ersten Stufe, beispielsweise 0,05–0,125 mg d-Norgestrel, und in der dritten Stufe 9–11 Dragées, die jeweils eine Östrogenmenge enthalten, die grösser ist als oder genau so gross ist wie die in der ersten Stufe und kleiner als oder genau so gross wie die in der zweiten Stufe, beispielsweise 0,025–0,050 mg Ethinylöstradiol, und eine Gestagenmenge, die grösser ist als die in der zweiten Stufe, aber nicht grösser als dreimal so gross wie die in der ersten Stufe, beispielsweise 0,10–0,25 mg d-Norgestrel, und gegebenenfalls in der vierten Stufe 7 Dragées ohne Östrogen und ohne Gestagen. Die Anzahl der Dosierungseinheiten in den drei östrogen- und gestagenhaltigen Stufen beträgt 21; zur Anpassung an den 28tägigen Zyklus können zu den 21 wirkstoffhaltigen Einheiten noch 7 wirkstofffreie Einheiten hinzugefügt werden.

Ein Zwei- und ein Dreistufenpräparat gemäss DE-A-2 365 103 befinden sich im Handel (B. Helwig: Moderne Arzneimittel, Stuttgart 1980, S. 1406–1407).

Im Vergleich mit den bekannten Kombinationspräparaten für die zyklische oder sequentiale Anwendung werden mit den Mehrstufenpräparaten höhere Verträglichkeit und bessere Zykluskontrolle erreicht.

In der europäischen Patentanmeldung Nr. 81 200 240 (Veröffentlichungsnummer 36 229) wird eine Variante eines Mehrstufenpräparats beschrieben. Diese Variante ist dadurch gekennzeichnet, dass in Einheiten der ersten Stufe eine höhere Östrogenmenge enthalten ist als in den Einheiten der folgenden Stufen. In der DE-A-2 431 704 werden Dreistufenpräparate mit 3 gleich langen Stufen beschrieben.

In den Mehrstufenkombinationspräparaten können die Östrogene in kleineren Mengen als 0,05 mg Ethinylöstradiol eingesetzt werden. Aufgrund des stufenweisen Aufbaus können auch die Gestagenmengen sehr niedrig gehalten werden. Auf diese Weise entstehen Kontrazeptiva mit den bislang geringsten Hormonmengen.

Es wurde nun gefunden, dass die Gestagenmenge in Mehrstufenkombinationspräparaten noch weiter gesenkt werden kann, wenn man als Gestagen das Gestoden (17• -Ethinyl-17• -hydroxy-18-methyl-östra-4,15-dien-3-on) verwendet. Die Östrogenmenge kann auf dem sehr niedrigen Niveau bekannter Dreistufenpräparate gehalten werden. Völlig unerwartet wird trotz Dosisreduzierung eine ausgezeichnete Zykluskontrolle bei guter Verträglichkeit erreicht. Die kontrazeptive Sicherheit ist immer gewährleistet.

Die vorliegende Erfindung betrifft somit ein Mehrstufenkombinationspräparat für die orale Kontrazeption aus 21 bzw. 28 Applikationseinheiten für tägliche Applikation einer Einheit, wobei in einer ersten Stufe von 4–6 Einheiten jede Einheit ein Östrogen in geringer Dosis und ein Gestagen in geringer Dosis und in einer zweiten Stufe von 4–6 Einheiten jede Einheit ein Östrogen mit gleicher oder gering angehobener, maximal bis auf das 2fache gesteigerter Dosis und ein Gestagen mit gleicher oder gering angehobener, maximal auf das 1 1/2fache gesteigerter Dosis und in einer dritten Stufe von 9–11 Einheiten jede Einheit ein Östrogen mit gleicher oder wieder gesenkter, maximal auf den Ausgangswert erniedrigter Dosis und ein Gestagen mit weiter angehobener, maximal auf das 3fache des Ausgangswertes gesteigerter Dosis enthält und die 3 Stufen zusammen aus 21 Einheiten bestehen und sich gegebenenfalls 7 weitere Einheiten ohne Östrogen und ohne Gestagen anschliessen, dadurch gekennzeichnet, dass das Gestagen Gestoden und das Östrogen Ethinylöstradiol ist und die Menge pro Einheit an Ethinylöstradiol in der ersten Stufe mindestens 0,02 mg und nicht mehr als 0,05 mg und an Gestoden in der ersten Stufe 0,04–0,07 mg beträgt.

Die Erfindung betrifft ferner die Verwendung des Mehrstufenkombinationspräparats zur oralen Kontrazeption. In der angegebenen Stufenfolge wird täglich eine Applikationseinheit verabreicht. Die Gesamtzahl der Tage, an denen eine Applikation der Wirkstoffkombinationen erfolgt, soll stets 21 betragen, daran schliessen sich 7 hormonfreie Tage an, an denen entweder täglich 1 Placebo oder keine Applikationseinheiten verabfolgt werden.

Die Applikationseinheiten sollen in den ersten 4–6 Tagen vorzugsweise 0,02–0,05 mg Ethinylöstradiol und 0,04–0,07 mg Gestoden pro Einheit enthalten. Die erfindungsgemäss in den 4–6 Tagen der zweiten Stufe eingesetzte Ethinylöstradiolmenge soll pro Einheit vorzugsweise 0,03–0,05 mg und die Gestodenmenge pro Einheit vorzugsweise 0,05–0,10 mg betragen. Die erfindungsgemäss in den 9–11 Tagen der dritten Stufe eingesetzte Ethinylöstradiolmenge soll pro Einheit vorzugsweise 0,02–0,05 mg und die Gestodenmenge pro Einheit vorzugsweise 0,08–0,12 mg betragen.

Ethinylöstradiol und Gestoden werden vorzugsweise zusammen oral appliziert, sie können jedoch auch getrennt und/oder parenteral verabreicht werden.

Ethinylöstradiol und Gestoden werden mit den in der galenischen Pharmazie üblichen Zusätzen, Trägersubstanzen und/oder Geschmackskorrigentien nach an sich bekannten Methoden zu den üblichen Applikationsformen verarbeitet. Für die bevorzugte orale Applikation kommen insbesondere Tabletten, Dragées, Kapseln, Pillen, Suspensionen oder Lösungen infrage.

Die 21 wirkstoffhaltigen Applikationseinheiten können durch 7 wirkstofffreie Applikationseinheiten (Placebos) ergänzt werden, um die Tage zu überbrücken, an denen keine Hormone appliziert werden sollen. Auf diese Weise wird die Gewohnheit, jeden Tag eine Einheit einzunehmen, beibehalten. Es ist dann nur noch notwendig, nach 28 Tagen (nach der Abbruchblutung) mit einer neuen Tablettenpackung fortzufahren.

Die Wirkstoffe können auch in Folienmaterial inkorporiert sein. Durch Unterteilung der Folienschicht können Applikationseinheiten mit entsprechender Dosierung für die buccale oder sublinguale Applikation bereitgestellt werden.

Gegenstand der Erfindung sind somit auch pharmazeutische Packungen, die dadurch gekennzeichnet sind, dass sie Mehrstufenkombinationspräparate in 21 oder 28 Applikationseinheiten in abgestimmter, festgelegter Reihenfolge enthalten, wobei die Reihenfolge den Stufen der täglichen Verabfolgung entspricht. Zweckmässigerweise unterscheiden sich die Placebos und die Applikationseinheiten der drei Stufen in ihrer Farbe oder Form.

Die pharmazeutische Packung kann unter anderem in Form einer Klarsichtpackung mit zum Beispiel 6 Dragées der ersten Stufe, 5 Dragées der zweiten Stufe, 10 Dragées der dritten Stufe und gegebenenfalls 7 Placebos ausgeführt werden, die jeweils täglich, also über 21 oder 28 Tage entnommen werden können.

Die erste und zweite Stufe können auch zu einer Stufe von 11 Dragées zusammengefasst werden.

Beispiel 1 (Zusammensetzung von Dragées)

| 1. Stufe (6 Dragées) | | |
|---|---|---|
| | 0,030 mg | Ethinylöstradiol |
| | 0,050 mg | Gestoden |
| | 37,455 mg | Lactose |
| | 15,500 mg | Maisstärke |
| | 0,065 mg | Ca-di-Na-edetat |
| | 1,700 mg | Kollidon 25 |
| | 0,200 mg | Magnesiumstearat |
| | 55,000 mg | Gesamtgewicht, das mit üblicher Zuckermischung und gegebenenfalls Farbstoff auf 90,000 mg ergänzt wird. |

| 2. Stufe (5 Dragées) | | |
|---|---|---|
| | 0,040 mg | Ethinylöstradiol |
| | 0,070 mg | Gestoden |
| | 37,425 mg | Lactose |
| | 15,500 mg | Maisstärke |
| | 0,065 mg | Ca-di-Na-edetat |
| | 1,700 mg | Kollidon 25 |
| | 0,200 mg | Magnesiumstearat |
| | 55,000 mg | Gesamtgewicht, das mit üblicher Zuckermischung und gegebenenfalls Farbstoff auf 90,000 mg ergänzt wird. |

| 3. Stufe (10 Dragées) | | |
|---|---|---|
| | 0,030 mg | Ethinylöstradiol |
| | 0,100 mg | Gestoden |
| | 37,405 mg | Lactose |
| | 15,500 mg | Maisstärke |
| | 0,065 mg | Ca-di-Na-edetat |
| | 1,700 mg | Kollidon 25 |
| | 0,200 mg | Magnesiumstearat |
| | 55,000 mg | Gesamtgewicht, das mit üblicher Zuckermischung und gegebenenfalls Farbstoff auf 90,000 mg ergänzt wird. |

Beispiel 2 (Zusammensetzung von Dragées)

| 1. Stufe (11 Dragées) | | |
|---|---|---|
| | 0,030 mg | Ethinylöstradiol |
| | 0,050 mg | Gestoden |
| | 37,455 mg | Lactose |
| | 15,500 mg | Maisstärke |
| | 0,065 mg | Ca-di-Na-edetat |
| | 1,700 mg | Kollidon 25 |
| | 0,200 mg | Magnesiumstearat |
| | 55,000 mg | Gesamtgewicht, das mit üblicher Zuckermischung und gegebenenfalls Farbstoff auf 90,000 mg ergänzt wird. |

| 2. Stufe        | 0,030 mg  | Ethinylöstradiol |
|-----------------|-----------|------------------|
| (10 Dragées)    | 0,100 mg  | Gestoden         |
|                 | 37,405 mg | Lactose          |
|                 | 15,500 mg | Maisstärke       |
|                 | 0,065 mg  | Ca-di-Na-edetat  |
|                 | 1,700 mg  | Kollidon 25      |
|                 | 0,200 mg  | Magnesiumstearat |
|                 | 55,000 mg | Gesamtgewicht, das mit üblicher Zucker- |

mischung und gegebenenfalls Farbstoff auf 90,000 mg ergänzt wird.

Klinische Untersuchungen zur Verträglichkeit und kontrazeptiven Sicherheit

Das Dreistufenpräparat gemäss Beispiel vorliegender Erfindung (A) wurde verglichen mit dem Dreistufenpräparat gemäss Beispiel der DE-B-23 65 103 (B).

Mit dem Prüfpräparat A wurden 377 Frauen im fortpflanzungsfähigen Alter in 2123 Zyklen und mit dem Prüfpräparat B 362 Frauen im fortpflanzungsfähigen Alter in 2088 Zyklen behandelt. Jede Frau erhielt 21 Tage lang täglich 1 Dragée, die anschliessenden 7 Tage, während der die Abbruchblutung erfolgte, blieben einnahmefrei. Diese Verabreichungsform wurde über 6 Zyklen beibehalten. Die meisten Frauen nahmen bis zum Ende an der Prüfung teil.

In der gesamten Behandlungszeit traten keine Schwangerschaften auf.

Beide Präparate waren gut verträglich.

Bei dem Dreistufenpräparat A traten weniger Zwischenblutungen auf als bei dem Dreistufenpräparat B.

| Zyklus            |   | 1.   | 3.   | 6.  |
|-------------------|---|------|------|-----|
| Schmierblutungen  | A | 11,8 | 7,4  | 3,1 |
|                   | B | 15,9 | 11,9 | 5,9 |
| Durchbruchblutungen | A | 2,0 | 1,5 | 0,9 |
|                   | B | 0,9  | 0,9  | 0,9 |
| Schmier- und Durch- | A | 1,1 | 0,9 | 0,3 |
| bruchblutungen    | B | 2,9  | 1,8  | 1,2 |

Die gute Zykluskontrolle bei A ist überraschend, da A im Gestagenanteil niedriger dosiert ist als B.

**Patentansprüche für die Vertragsstaaten BE, CH, LI, DE, FR, GB, IT, LU, NL, SE**

1. Zwei- bzw. Dreistufenkombinationspräparat für die orale Kontrazeption aus 21 bzw. 28 Applikationseinheiten für tägliche Applikation einer Einheit, wobei in einer ersten Stufe von 4–6 Einheiten jede Einheit ein Östrogen in geringer Dosis und ein Gestagen in geringer Dosis und in einer zweiten Stufe von 4–6 Einheiten jede Einheit ein Östrogen mit gleicher oder gering angehobener, maximal bis auf das 2fache gesteigerter Dosis und ein Gestagen mit gleicher oder gering angehobener, maximal auf das 1 1/2fache gesteigerter Dosis und in einer dritten Stufe von 9–11 Einheiten jede Einheit ein Östrogen mit gleicher oder wieder gesenkter, maximal auf den Ausgangswert erniedrigter Dosis und ein Gestagen mit weiter angehobener, maximal auf das 3fache des Ausgangswertes gesteigerter Dosis enthält und die 3 Stufen zusammen aus 21 Einheiten bestehen und sich gegebenenfalls 7 weitere Einheiten ohne Östrogen und ohne Gestagen anschliessen, dadurch gekennzeichnet, dass das Gestagen Gestoden und das Östrogen Ethinylöstradiol ist, wobei in jeder Applikationseinheit der ersten Stufe die Menge an Ethinylöstradiol mindestens 0,02 mg und nicht mehr als 0,05 mg und die Menge an Gestoden 0,04–0,07 mg beträgt.

2. Präparat nach Anspruch 1, dadurch gekennzeichnet, dass die Anzahl der Applikationseinheiten in der ersten Stufe 6, in der zweiten Stufe 5 und in der dritten Stufe 10 beträgt.

3. Präparat nach Anspruch 1, dadurch gekennzeichnet, dass die Applikationseinheiten Tabletten oder Dragées darstellen.

4. Präparat nach Anspruch 1, dadurch gekennzeichnet, dass die erste Stufe aus 6 Dragées besteht, wobei jedes Dragée 0,03 mg Ethinylöstradiol und 0,05 mg Gestoden enthält, und die zweite Stufe aus 5 Dragées besteht, wobei jedes Dragée 0,04 mg Ethinylöstradiol und 0,07 mg Gestoden enthält, und die dritte Stufe aus 10 Dragées besteht, wobei jedes Dragée 0,03 mg Ethinylöstradiol und 0,10 mg Gestoden enthält.

5. Zwei- bzw. Dreistufenkombinationspräparat gemäss Anspruch 1 zur Verwendung bei der oralen Kontrazeption.

6. Verfahren zur Herstellung eines Zwei- bzw. Dreistufenkombinationspräparats gemäss Anspruch 1, dadurch gekennzeichnet, dass man Ethinylöstradiol und Gestoden in den jeweils angegebenen Mengen mit den in der galenischen Pharmazie üblichen Zusätzen, Trägersubstanzen und/oder Geschmackskorrigentien vermischt, in eine geeignete Applikationsform überführt und in dem zur Verabreichung geeigneten System verpackt, wobei 21 oder 28 Applikationseinheiten so angeordnet sind, dass ihre Reihenfolge den Stufen der täglichen Verabfolgung entspricht.

7. Verfahren nach Anspruch 6, dadurch gekennzeichnet, dass die Applikationseinheiten in der ersten Stufe 0,02–0,05 mg Ethinylöstradiol und 0,04–0,07 mg Gestoden, in der zweiten Stufe 0,03–0,05 mg Ethinylöstradiol und 0,05–0,10 mg Gestoden und in der dritten Stufe 0,02–0,05 mg Ethinylöstradiol und 0,08–0,12 mg Gestoden enthalten.

8. Verfahren nach Anspruch 6, dadurch gekennzeichnet, dass die Anzahl der Applikationseinheiten in der ersten Stufe 6, in der zweiten Stufe 5 und in der dritten Stufe 10 beträgt.

4

9. Verfahren nach Ansrpuch 6, dadurch gekennzeichnet, dass die Applikationseinheiten Tabletten oder Dragées darstellen.

10. Verfahren nach Anspruch 6, dadurch gekennzeichnet, dass die erste Stufe aus 6 Dragées besteht, wobei jedes Dragée 0,03 mg Ethinylöstradiol und 0,05 mg Gestoden enthält, und die zweite Stufe aus 5 Dragées besteht, wobei jedes Dragée 0,04 mg Ethinylöstradiol und 0,07 mg Gestoden enthält, und die dritte Stufe aus 10 Dragées besteht, wobei jedes Dragée 0,03 mg Ethinylöstradiol und 0,10 mg Gestoden enthält.

## Patentansprüche für den Vertragsstaat AT

1. Verfahren zur Herstellung eines Zwei- bzw. Dreistufenkombinationspräparats für die orale Kontrazeption aus 21 bzw. 28 Applikationseinheiten für tägliche Applikation einer Einheit, wobei in einer ersten Stufe von 4–6 Einheiten jede Einheit ein Östrogen in geringer Dosis und ein Gestagen in geringer Dosis und in einer zweiten Stufe von 4–6 Einheiten jede Einheit ein Östrogen mit gleicher oder gering angehobener, maximal bis auf das 2fache gesteigerter Dosis und ein Gestagen mit gleicher oder gering angehobener, maximal auf das 1 1/2fache gesteigerter Dosis und in einer dritten Stufe von 9–11 Einheiten jede Einheit ein Östrogen mit gleicher oder wieder gesenkter, maximal auf den Ausgangswert erniedrigter Dosis und ein Gestagen mit weiter angehobener, maximal auf das 3fache des Ausgangswertes gesteigerter Dosis enthält und die 3 Stufen zusammen aus 21 Einheiten bestehen und sich gegebenenfalls 7 weitere Einheiten ohne Östrogen und ohne Gestagen anschliessen, mit der Massgabe, dass das Gestagen Gestoden und das Östrogen Ethinylöstradiol ist, wobei in jeder Applikationseinheit der ersten Stufe die Menge an Ethinylöstradiol mindestens 0,02 mg und nicht mehr als 0,05 mg und die Menge an Gestoden 0,04–0,07 mg beträgt, dadurch gekennzeichnet, dass man Ethinylöstradiol und Gestoden in den jeweils angegebenen Mengen mit den in der galenischen Pharmazie üblichen Zusätzen, Trägersubstanzen und/oder Geschmackskorrigentien vermischt, in eine geeignete Applikationsform überführt und in dem zur Verabreichung geeigneten System verpackt, wobei 21 oder 28 Applikationseinheiten so angeordnet sind, dass ihre Reihenfolge den Stufen der täglichen Verabfolgung entspricht.

2. Verfahren nach Anspruch 1, dadurch gekennzeichnet, dass die Applikationseinheiten in der ersten Stufe 0,02–0,05 mg Ethinylöstradiol und 0,04–0,07 mg Gestoden, in der zweiten Stufe 0,03–0,05 mg Ethinylöstradiol und 0,05–0,10 mg Gestoden und in der dritten Stufe 0,02–0,05 mg Ethinylöstradiol und 0,08–0,12 mg Gestoden enthalten.

3. Verfahren nach Anspruch 1, dadurch gekennzeichnet, dass die Anzahl der Applikationseinheiten in der ersten Stufe 6, in der zweiten Stufe 5 und in der dritten Stufe 10 beträgt.

4. Verfahren nach Anspruch 1, dadurch gekennzeichnet, dass die Applikationseinheiten Tabletten oder Dragées darstellen.

5. Verfahren nach Anspruch 1, dadurch gekennzeichnet, dass die erste Stufe aus 6 Dragées besteht, wobei jedes Dragée 0,03 mg Ethinylöstradiol und 0,05 mg Gestoden enthält, und die zweite Stufe aus 5 Dragées besteht, wobei jedes Dragée 0,04 mg Ethinylöstradiol und 0,07 mg Gestoden enthält, und die dritte Stufe aus 10 Dragées besteht, wobei jedes Dragée 0,03 mg Ethinylöstradiol und 0,10 mg Gestoden enthält.

## Revendications pour les Etats contractants BE, CH, DE, FR, GB, IT, LI, LU, NL, SE

1. Préparation combinée biphasique ou triphasique pour la contraception orale, comprenant 21 ou 28 unités de prise pour l'administration quotidienne d'une unité, à savoir, dans une première phase, de 4 à 6 unités renfermant chacune une faible dose d'estrogène et une faible dose de progestatif, dans une deuxième phase de 4 à 6 unités renfermant chacune un estrogène en une dose égale ou légèrement supérieure, au plus double, et un progestatif en une dose égale ou légèrement supérieure, au plus en une dose multipliée par 1,5, et, dans une troisième phase, de 9 à 11 unités renfermant chacune un estrogène à une dose égale ou à nouveau diminuée, abaissée au maximum à la valeur de départ, et un progestatif à une dose encore augmentée, élevée au maximum à trois fois la valeur de départ, les trois phases étant constituées de 21 unités et étant éventuellement suivies de 7 unités supplémentaires sans estrogène ni progestatif, cette préparation combinée multiphasique étant caractérisée en ce que le gestagène est le gestodène et l'estrogène est l'éthynyl-estradiol, chaque unité de prise de la première phase contenant de 0,02 à 0,05 mg d'éthynyl-estradiol et de 0,04 à 0,07 mg de gestodène.

2. Préparation selon la revendication 1 caractérisée en ce que le nombre des unités de prise est de 6 dans la première phase, de 5 dans la deuxième et de 10 dans la troisième.

3. Préparation selon la revendication 1 caractérisée en ce que les unités de prise sont des comprimés ou des dragées.

4. Préparation selon la revendication 1 caractérisée en ce que la première phase est constituée de 6 dragées contenant chacune 0,03 mg d'éthynyl-estradiol et 0,05 mg de gestodène, la deuxième phase de 5 dragées contenant chacune 0,04 mg d'éthynyl-estradiol et 0,07 mg de gestodène, et la troisième phase de 10 dragées contenant chacune 0,03 mg d'éthynyl-estradiol et 0,10 mg de gestodène.

5. Préparation combinée biphasique ou triphasique selon la revendication 1 pour l'application dans la contraception orale.

6. Procédé pour confectionner une préparation combinée biphasique ou triphasique selon la revendication 1, procédé caractérisé en ce qu'on mélange l'éthynyl-estradiol et le gestodène en les quantités indiquées pour chacune de ces substances, avec des additifs, excipients et/ou correcteurs de goût pris parmi ceux dont on se sert habituellement en pharmacie, on met le mélange sous une forme d'administration appropriée et on l'emballe dans un système convenant pour l'administration, les unités de prise, au nombre de 21 ou de 28, étant disposées de telle façon que leur ordre de succession corresponde aux phases de l'administration journalière.

7. Procédé selon la revendication 6 caractérisée en ce que les unités de prise contiennent chacune, dans la première phase, de 0,02 à 0,05 mg d'éthynyl-estradiol et de 0,04 à 0,07 mg de gestodène, dans la seconde phase de 0,03 à0,05 mg d'éthynyl-estradiol et de 0,05 à 0,10 mg de gestodène et, dans la troisième phase, de 0,02 à 0,05 mg d'éthynyl-estradiol et de 0,08 à 0,12 mg de gestodène.

8. Procédé selon la revendication 6 caractérisé en ce que le nombre des unités de prise est de 6 dans la première phase, de 5 dans la deuxième et de 10 dans la troisième.

9. Procédé selon la revendication 6 caractérisé en ce que les unités de prise sont des comprimés ou des dragées.

10. Procédé selon la revendication 6 caractérisé en ce que la première phase est constituée de 6 dragées contenant chacune 0,03 mg d'éthynyl-estradiol et 0,05 mg de gestodène, la seconde phase de 5 dragées contenant chacune 0,04 mg d'éthynyl-estradiol et 0,07 mg de gestodène, et la troisième phase de 10 dragées contenant chacune 0,03 mg d'éthynyl-estradiol et 0,10 mg de gestodène.

**Revendications pour l'Etat contractant AT**

1. Procédé pour confectionner une préparation combinée biphasique ou triphasique pour la contraception orale, comprenant 21 ou 28 unités de prise pour l'administration quotidienne d'une unité, à savoir, dans une première phase, de 4 à 6 unités renfermant chacune une faible dose d'estrogène et une faible dose de progestatif, dans une deuxième phase de 4 à 6 unités renfermant chacune un estrogène en une dose égale ou légèrement supérieure, au plus double, et un progestatif en une dose égale ou légèrement supérieure, au plus en une dose multipliée par 1,5, et, dans une troisième phase, de 9 à 11 unités renfermant chacune un estrogène en une dose égale ou à nouveau diminuée, abaissée au maximum à la valeur de départ, et un progestatif en une dose encore augmentée, élevée au maximum à trois fois la valeur de départ, les trois phases étant constituées de 21 unités et étant éventuellement suivies de 7 unités supplémentaires sans estrogène ni progestatif, avec la condition que le progestatif soit le gestodène et que l'estrogène soit l'éthynyl-estradiol et que chaque unité de prise de la première phase contienne de 0,02 à 0,05 mg d'éthynyl-estradiol et de 0,04 à 0,07 mg de gestodène, procédé caractérisé en ce qu'on mélange l'éthynyl-estradiol et le gestodène en les quantités indiquées pour chacune de ces substances, avec des additifs, excipients et/ou correcteurs de goût pris parmi ceux dont on se sert habituellement en pharmacie, on met le mélange sous une forme d'administration appropriée et on l'emballe dans un système convenant pour l'administration, les unités de prise, au nombre de 21 ou de 28, étant disposées de telle façon que leur ordre de succession corresponde aux phases de l'administration journalière.

2. Procédé selon la revendication 1 caractérisé en ce que les unités de prise contiennent chacune, dans la première phase, de 0,02 à 0,05 mg d'éthynyl-estradiol et de 0,04 à 0,07 mg de gestodène, dans la seconde phase de 0,03 à 0,05 mg d'éthynyl-estradiol et de 0,05 à 0,10 mg de gestodène et, dans la troisième phase, de 0,02 à 0,05 mg d'éthynyl-estradiol et de 0,08 à 0,12 mg de gestodène.

3. Procédé selon la revendication 1 caractérisé en ce que le nombre des unités de prise est de 6 dans la première phase, de 5 dans la deuxième et de 10 dans la troisième.

4. Procédé selon la revendication 1 caractérisé en ce que les unités de prise sont des comprimés ou des dragées.

5. Procédé selon la revendication 1 caractérisé en ce que la première phase est constituée de 6 dragées contenant chacune 0,03 mg d'éthynyl-estradiol et 0,05 mg de gestodène, la seconde phase de 5 dragées contenant chacune 0,04 mg d'éthynyl-estradiol et 0,07 mg de gestodène, et la troisième phase de 10 dragées contenant chacune 0,03 mg d'éthynyl-estradiol et 0,10 mg de gestodène.

**Claims for the contracting states BE, CH, LI, DE, FR, GB, IT, LU, NL, SE**

1. Two- or three-phase combined preparation for oral contraception made up of 21 or 28 units of administration for daily administration of one unit wherein, in a first phase of 4–6 units, each unit contains an oestrogen in a low dose and a gestagen in a low dose and, in a second phase of 4–6 units, each unit contains an oestrogen of the same dose or a slightly increased dose, increased to a maximum of twice, and a gestagen of the same or a slightly increased dose, increased to a maximum of one and a half times and, in a third phase of 9–11 units, each unit contains an oestrogen of the same dose or a dose reduced again, the maximum reduction being to the initial value, and a gestagen of a further increased dose, increased to a maximum of three times the initial value, and the three phases together consist of 21 units, optionally followed by seven further units without oestrogen and without gestagen, characterised in that the gestagen is gestodene and the oestrogen is ethynyloestradiol, wherein, in each unit of administration of the first phase, the amount of ethynyloestradiol is at least 0,02 mg and not more than 0,05 mg and the amount of gestodene is 0,04–0,07 mg.

2. Preparation according to claim 1, characterised in that the number of units of administration in the first phase is 6, in the second phase 5, and in the third phase 10.

3. Preparation according to claim 1, characterised in that the units of administration are tablets or dragées.

4. Preparation according to claim 1, characterised in that the first phase consists of 6 dragées, each dragée containing 0,03 mg of ethynyloestradiol and 0,05 mg of gestodene, and the second phase consists of 5 dragées, each dragée containing 0,04 mg of ethynyloestradiol and 0,07 mg of gestodene, and the third phase consists of 10 dragées, each dragée containing 0,03 mg of ethynyloestradiol and 0,10 mg of gestodene.

5. Two- or three-phase combined preparation according to claim 1 each for use in oral contraception.

6. Process for the preparation of a two- or three-phase combined preparation according to claim 1, characterised in that ethynyloestradiol and gestodene are mixed, in the respective amounts specified, with additives, carriers and/or flavouring substances customary in galenical pharmacy, converted into a suitable form of administration and packed in a system suitable for administration, 21 or 28 units of administration being so arranged that their sequence corresponds to the phases of the daily administration.

7. Process according to claim 6, characterised in that the units of administration in the first phase contain 0,02–0,05 mg of ethynyloestradiol and 0,04–0,07 mg of gestodene, in the second phase contain 0,03–0,05 mg of ethynyloestradiol and 0,05–0,10 mg of gestodene and in the third phase contain 0,02–0,05 mg of ethynyloestradiol and 0,08–0,12 mg of gestodene.

8. Process according to claim 6, characterised in that the number of units of administration is in the first phase 6, in the second phase 5 and in the third phase 10.

9. Process according to claim 6, characterised in that the units of administration are tablets or dragées.

10. Process according to claim 6, characterised in that the first phase consists of 6 dragées, each dragée containing 0,03 mg of ethynyloestradiol and 0,05 mg of gestodene, and the second phase consists of 5 dragées, each dragée containing 0,04 mg of ethynyloestradiol and 0,07 mg of gestodene, and the third phase consists of 10 dragées, each dragée containing 0,03 mg of ethynyloestradiol and 0,10 mg of gestodene.

**Claims for the contracting state AT**

1. Process for the preparation of a two- or three-phase combined preparation for oral contraception made up of 21 or 28 units of administration for daily administration of one unit wherein, in a first phase of 4–6 units, each unit contains an oestrogen in a low dose and a gestagen in a low dose and, in a second phase of 4–6 units, each unit contains an oestrogen of the same dose or a slightly increased dose, increased to a maximum of twice, and a gestagen of the same or a slightly increased dose, increased to a maximum of one and a half times and, in a third phase of 9–11 units, each unit contains an oestrogen of the same dose or a dose reduced again, the maximum reduction being to the initial value, and a gestagen of a further increased dose, increased to a maximum of three times the initial value, and the three phases together consist of 21 units, optionally followed by seven further units without oestrogen and without gestagen, with the proviso that the gestagen is gestodene and the oestrogen is ethynyloestradiol, wherein, in each unit of administration of the first phase, the amount of ethynyloestradiol is at least 0,02 mg and not more than 0,05 mg and the amount of gestodene is 0,04–0,07 mg, characterised in that ethynyloestradiol and gestodene are mixed, in the respective amounts specified, with additives, carriers and/or flavouring substances customary in galenical pharmacy, converted into a suitable form of administration and packed in a system suitable for administration, 21 or 28 units of administration being so arranged that their sequence corresponds to the phases of the daily administration.

2. Process according to claim 1, characterised in that the units of administration in the first phase contain 0,02–0,05 mg of ethynyloestradiol and 0,04–0,07 mg of gestodene, in the second phase contain 0,03–0,05 mg of ethynyloestradiol and 0,05–0,10 mg of gestodene and in the third phase contain 0,02–0,05 mg of ethynyloestradiol and 0,08–0,12 mg of gestodene.

3. Process according to claim 1, characterised in that the number of units of administration is in the first phase 6, in the second phase 5 and in the third phase 10.

4. Process according to claim 1, characterised in that the units of administration are tablets or dragées.

5. Process according to claim 1, characterised in that the first phase consists of 6 dragées, each dragée containing 0,03 mg of ethynyloestradiol and 0,05 mg of gestodene, and the second phase consists of 5 dragées, each dragée containing 0,04 mg of ethynyloestradiol and 0,07 mg of gestodene, and the third phase consists of 10 dragées, each dragée containing 0,03 mg of ethynyloestradiol and 0,10 mg of gestodene.